# EUROPEAN PATENT SPECIFICATION

(11) **EP 1 525 859 B1**
(45) Date of publication and mention of the grant of the patent: **02.04.2008**
(21) Application number: 04256536.6
(22) Date of filing: 22.10.2004
(51) Int. Cl.: A61F 2/82

(54) **Self-expanding stent and stent delivery system for treatment of vascular disease**
Selbstexpandierbarer Stent und Stenteinbringungssystem zur Behandlung von Gefässerkrankungen
Stent auto-expansible et système de pose de stent pour le traitement d'une maladie vasculaire

(30) Priority: 23.10.2003 US 691846
(43) Date of publication of application: 27.04.2005
(73) Proprietor: Cordis Neurovascular, Inc., Miami Lakes, Florida 33014 (US)
(72) Inventor: Jones, Donald K., Lauderhill Florida 33351 (US); Mitelberg, Vladimir, Aventura Florida 33180 (US)
(74) Representative: Mercer, Christopher Paul

(56) References cited:
- EP-A- 1 374 801
- US-A1- 2001 051 822
- US-A1- 2002 016 597
- US-A1- 2002 151 956

## Description

### Background of the Invention

### Field of the Invention

This invention relates to intravascular stents and stent delivery systems for treating a stenosis or for covering an aneurysm within a blood vessel. More particularly, this invention relates to a very small, self-expanding stent which may be used for percutaneous transluminal angioplasty of occluded blood vessels or for providing a cover for an aneurysm within the brain of a patient.

### Description of the Prior Art

On a worldwide basis, nearly one million balloon angioplasties are performed annually to treat vascular diseases such as blood vessels that are clogged or narrowed by a lesion or stenosis. The objective of this procedure is to increase the inner diameter of the partially occluded blood vessel lumen. In an effort to prevent restenosis without requiring surgery, short flexible cylinders or scaffolds, referred to as stents, are often placed into the blood vessel at the site of the stenosis.

Stents are typically made of metal or polymers and are widely used for reinforcing diseased blood vessels. Some stents are expanded to their proper size using a balloon catheter. Such stents are referred to as ''balloon expandable stents". Other stents, referred to as "self-expanding stents", are designed to automatically expand when released. Both balloon expandable stents and self-expanding stents are generally compressed onto a small diameter catheter and are deployed within a blood vessel.

Several types of balloon catheters have been disclosed in the prior art. One such balloon catheter is disclosed in U.S. Patent No. 5,843,090, entitled "Stent Delivery Device," which balloon catheter is used as a stent delivery device. U.S. Patent No. 5,639,274, entitled "Integrated Catheter System for Balloon Angioplasty and Stent Delivery," discloses an integrated catheter system including a stent deployment catheter and a balloon angioplasty catheter.

Recently, filters mounted on the distal end of guidewires have been proposed for intravascular blood filtration during balloon angioplasty and the delivery of vascular stents. One such filter is disclosed in U.S. Patent No. 6,168,579, entitled "Filter Flush System and Methods of Use." This patent discloses a filter flush system for temporary placement of a filter into a blood vessel. The filter system includes a guidewire for carrying an expandable filter which is collapsed to pass through the lumen of a guiding catheter and is then be expanded upstream of a stenosis prior to angioplasty or to the placement of a stent. U.S. Patent Application Publication No. 2002/0115942, entitled "Low Profile Emboli Capture Device," discloses an emboli capture device comprised of a filter and a self-expanding stent. The self-expanding stent is attached to the filter in order to open the filter when the emboli capture device is placed within an artery.

Self-expanding stents have generally been compressed onto the outer circumference of a delivery catheter and are then held in the compressed state by an outer catheter which surrounds both the delivery catheter and the stent. As the compressed stent is moved distally out of the distal end of the outer catheter, the stent begins expanding and expands until contact is made between the outer surface of the stent and the inner surface of the wall of a vessel. One problem with a self-expanding stent of this type is that once the stent has expanded within the vessel, it is very difficult to remove the stent or to reposition the stent to a different location within the vessel.

Another form of a self-expanding stent and delivery system is disclosed in European Patent Application No. 04255121.8, entitled, "Self-Expanding Stent And Stent Delivery System With Distal Protection," and European Patent Application No. 04255205.9,
entitled, "Self-Expanding Stent And Stent Delivery System For Treatment Of Vascular Stenosis." Said documents fall under the requirements of Art. 54(3) EPC. Both of these patent applications disclose a stent and stent delivery system in which the distal end of the stent may be expanded into contact with the vessel wall by partially withdrawing the outer catheter from the stent. The proximal end of the stent may be maintained in a compressed form. If it is then determined that the distal end of the stent is properly positioned within the vessel, the outer catheter may be completely withdrawn permitting the proximal end of the stent to expand into contact with the vessel wall. On the other hand, if after the distal end of the stent is expanded into contact with the vessel wall it is determined that the stent should be withdrawn, or repositioned, the outer catheter may be moved distally back over the distal end of the stent to cause the distal end of the stent to again become compressed within the outer catheter. The stent, contained within the outer catheter, may be repositioned to a different location within the vessel and the same procedure may again be followed for positioning the stent at the new location.

U.S. 2002/151956 A1 discloses a self-expanding stent and stent delivery system having an elongated core member, a self-expanding stent mounted and compressed onto the elongated core member, and an actuable retaining ring disposed around the self-expanding stent.

### Summary of the Invention

In accordance with the present invention, there is provided a self-expanding stent and stent delivery system as recited in the claims. The stent delivery system may include an elongated delivery catheter having a lumen extending therethrough. Disposed within the lumen of the delivery catheter may be the elongated core member. An actuatable retaining ring member may be placed around the distal end of the self-expanding stent and served to hold the distal end of the stent in its compressed state. Upon actuation, such as by heating, the retaining ring member yields thereby releasing the distal end of the compressed stent with the result that the stent expands into contact with the inside wall of a blood vessel.

The retaining ring which is disposed about the proximal portion of the self-expanding stent serves to retain the proximal portion of the stent thereby preventing this portion of the stent to be expanded until such time as the retaining ring is actuated.

The actuatable retaining rings may be formed of a material which when heated yields to thereby become severed in order to permit the compressed, self-expanded stent to expand into contact with the walls of a vessel.

The stent delivery system may include a heating element positioned in proximity to the actuatable retaining rings and may also include electrical conductors connected to the heating element for, upon being energized, causing the heating element to heat the actuatable retaining rings with the result that the retaining rings become severed to permit the self-expanding stent to expand into contact with the walls of a vessel.

The actuatable retaining rings may be comprised of a polymeric material, such as a hot melt polymer, and the heating element may take the form of a resistive heating element.

### Brief Description of the Drawings

Figure 1 is a partially sectional view of an integrated balloon catheter and a self-expanding stent mounted;
Figure 2 is an oblique view of the self-expanding stent of Figure 1 mounted on a core wire and held in a compressed state by two actuatable retaining rings;
Figure 2a is an oblique view of the two actuatable retaining rings and corresponding heating elements;
Figure 3 is a sectional view of the integrated balloon catheter and self-expanding stent of Figure 1 within a blood vessel prior to expansion of the balloon and the self-expanding stent;
Figure 4 is a sectional view of the integrated balloon catheter and self-expanding stent positioned within a blood vessel with the balloon fully expanded;
Figure 5 is a sectional view of the integrated balloon catheter and self-expanding stent with the balloon deflated and the outer catheter being moved proximally and with the distal actuatable retaining ring severed to release the stent thereby allowing the distal end of the stent to expand within the blood vessel;
Figure 6 is a sectional view of the proximal actuatable retaining ring severed thereby permitting the self-expanding stent to become fully expanded within the blood vessel;
Figure 7 is a sectional view of the balloon catheter and elongated core wire withdrawn proximally from the self-expanding stent; and,
Figure 8 is an elevational view of the self-expanding stent within the blood vessel with the balloon catheter and elongated core wire removed from the blood vessel.

### Description of the Preferred Embodiment

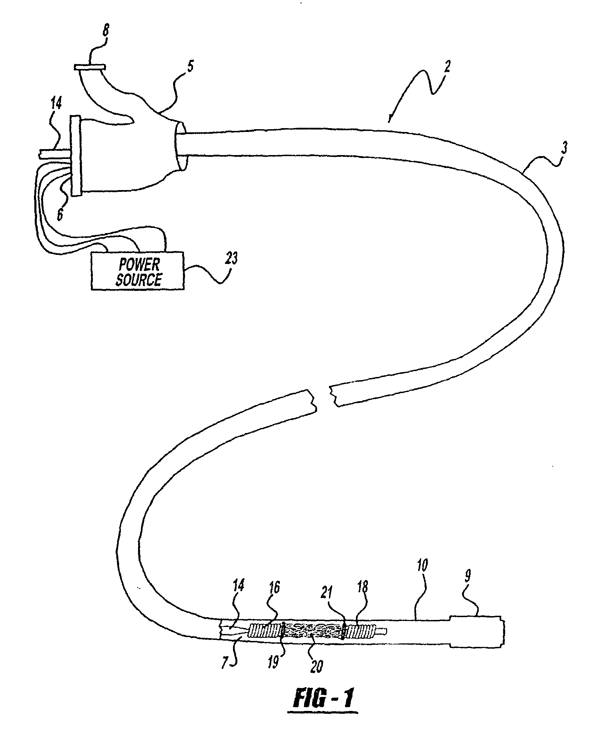

Figure 1 illustrates an integrated balloon catheter and self-expanding stent including a balloon catheter 2 comprised of an outer catheter 3 having a Luer connector coupling member 5. The coupling member 5 includes a delivery port 6 which communicates with a delivery lumen 7, which in turn, extends throughout the length of the balloon catheter 2. The coupling member 5 also includes an inflation port 8 used to inflate and expand the balloon 9 disposed about the distal portion 10 of the outer catheter 3. The balloon catheter 2 is sufficiently rigid to be pushed distally through a blood vessel, yet flexible enough to traverse the narrow and tortuous blood vessels within a blood vessel, such as a blood vessel within the brain.

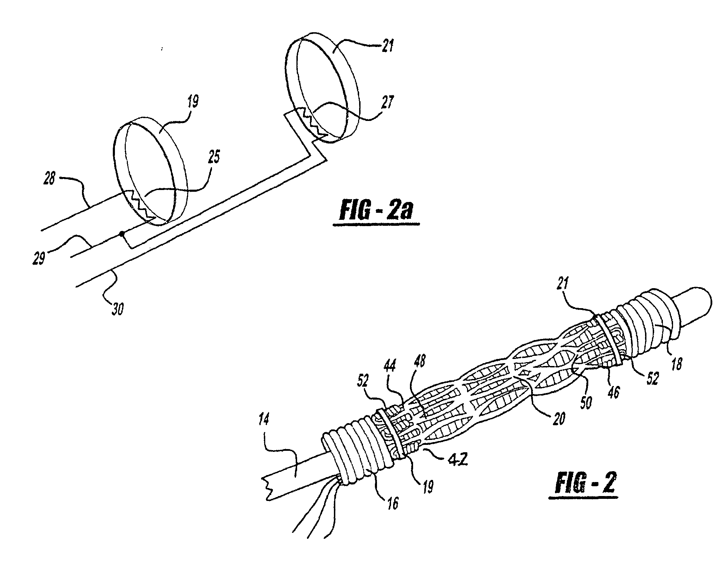

Slidably disposed within the delivery lumen 7 is an elongated core wire 14. Disposed about the elongated core wire 14 are a proximal cylindrical member 16 and a distal cylindrical member 18, both of which may take the form of a helical coil. A self-expanding sent 20 is mounted on the elongated core wire 14. The proximal and distal cylindrical members 16, 18 are spaced apart to form a gap between the cylindrical member and serve as stop members extending radially outward from the core wire 14 to engage the stent 20 in order to prevent longitudinal movement of the stent relative to the core wire 14. A proximal actuatable retaining ring 19 and a distal actuatable retaining ring 21 extend around the proximal and distal portions, respectively, of the stent 20 and serve to restrain the stent in a compressed state. The actuatable retaining rings are caused to yield and then sever upon the application of an electrical current which is applied through a power source 23. The construction and operation of the actuatable retaining rings 19, 21 are shown in more detail in Figures 2 and 2a.

Figure 2 illustrates the self-expanding stent 20 mounted on the elongated core wire 14. Disposed about the elongated core wire 14 is the proximal cylindrical member 16, which preferably takes the form of a helically wound flexible coil. The coil may be formed of metal or of a polymer material. A distal cylindrical member 18 is disposed about the elongated core wire 14 and is positioned distally from the proximal cylindrical member 16. The distal cylindrical member 18 is spaced apart from the proximal cylindrical member 16 such that the space between the proximal and distal cylindrical members 16, 18 forms a gap 42. The distal cylindrical member 18 is also preferably a helically wound flexible coil.

The self-expanding stent 20 may take on many different patterns or configurations. Examples of such self-expanding stents are disclosed in EP-A-1 266 639 and EP-A-1 266 640.

The stent 20 is preferably coated with a bioactive agent, such as heparin or rapamycin, to prevent restenosis within the vessel. Examples of such coatings are disclosed in U.S. Patent Nos. 5,288,711; 5,516,781; 5,563,146 and 5,646,160.

The self-expanding stent 20 is preferably laser cut from a nitinol tube to form a skeletal tubular member. The skeletal tubular member has a small diameter and a thin wall which defines a plurality of cells formed by a plurality of interconnected strut members. The nitinol is treated so as to exhibit superelastic properties at body temperature. Additionally, the stent 20 includes proximal and distal strut members 44, 46 coupled to the proximal and distal sections 48, 50 of the stent. The proximal and distal strut members 44, 46 are cut to form threads on the strut members during the laser-cutting of the stent 20. Radiopaque coils are then wound onto the threads of the proximal and distal strut members 44, 46 to form anchor members 52. Preferably, the stent 20 includes eight anchor members 52. When the self-expanding stent 20 is mounted on the elongated core wire 14, the anchor members 52 align with and are disposed within the first gap 42 thus coupling the stent to the elongated core wire 14. In this configuration, the stent 20 can be moved distally through the delivery lumen 7 of the balloon catheter 2 by moving the core wire 14 distally. The self-expanding stent 20 is described in more detail in U.S. Patent Application, Serial No. 10/608,659 (Attorney Docket No. CRD5001 CIP), entitled "Expandable Stent with Radiopaque Markers and Stent Delivery System," filed on June 27, 2003 and assigned to the same assignee as the present patent application.

The corresponding EP-Application 1400 219 falls under Art. 54(3) EPC.

Figures 2 and 2a illustrate in more detail the actuatable retaining rings 19, 21 which serve to hold the self-expanding stent in its compressed state. The actuatable retaining rings 19, 21 are preferably formed from a filament of a hot melt polymer, such as a polymer marketed by Minnesota Mining and Manufacturing under the trade name Jet Melt, Catalog No. 3783-TC, however, various other biocompatible thermoplastic polymers which exhibit the characteristic of melting, or decreasing yield strength when heat is applied, could be used for the rings. The application of heat to the polymer causes the polymer to exhibit the characteristic of yielding and ultimately severing to thereby release the compressed stent. The actuatable retaining rings 19,21 serve to clamp the stent onto the elongated core wire 14. Resistive heating elements 25, 27 are placed in proximity to the actuatable retaining rings 19, 21, respectively, and the heating elements are coupled through electrical conductors 28, 29, 30, to the power source 23. Accordingly, upon application of electrical current to the resistive heating element 27, the heating element 27 generates heat, which in turn, is applied to the corresponding distal actuatable retaining ring 21 causing this retaining ring to yield and then sever thereby releasing the distal portion of the compressed stent 20. Similarly, when electrical current is applied to the resistive heating element 25, this heating element begins to heat and causes the proximal actuatable retaining ring 19 to yield and sever, and in turn, it releases the proximal portion of the stent 20 to expand from its compressed state.

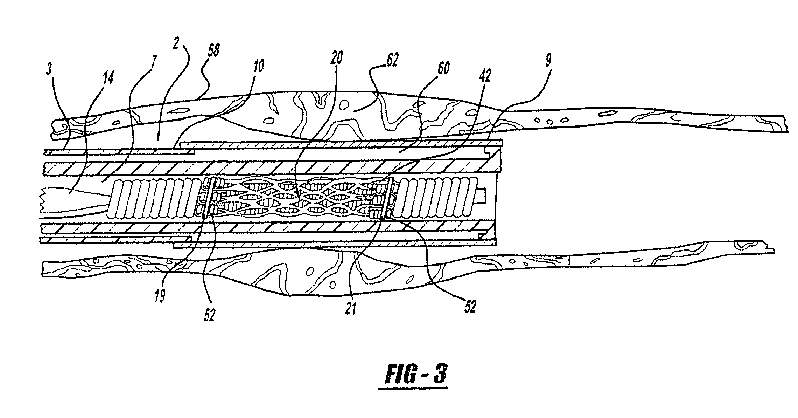

Figure 3 shows the balloon catheter 2 inserted within a blood vessel 58 of the brain of a patient. The balloon catheter 2 includes an expandable balloon 9 disposed about the distal portion 10 of the elongated outer catheter 3. In the preferred embodiment of the present invention, an inflation lumen 60 extends from the inflation port 8 and communicates with the balloon 9. To perform an angioplasty of the blood vessel 58, a fluid is injected into the inflation lumen 60, through the inflation port 8, to thus expand the balloon 9. The operation of the balloon catheter is described in more detail in U.S. Patent No. 6,585,687.

Typically, the balloon catheter 2 is advanced distally through the blood vessel 58 over a guidewire until it is aligned with a stenosis 62. Then, the guidewire is removed and the elongated core wire 14 is inserted into the delivery lumen 7 of the balloon catheter 2. The self-expanding stent 20 is mounted on the elongated core wire 14 such that the anchor members 52 align with and are disposed within the gap 42, between the proximal and distal cylindrical members 16, 18. In this configuration, the stent 20 is engaged to the core wire 14 so that the stent may be moved proximally and distally through the delivery lumen 7 of the balloon catheter 2.

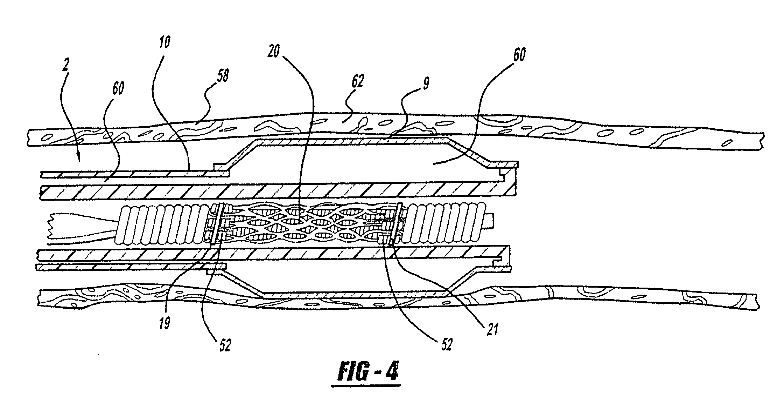

Figure 4 illustrates the balloon catheter 2 having the expandable balloon 9 fully expanded within the blood vessel 58. Preferably, the balloon 9 is expanded by injecting fluid into the inflation lumen 60 of the balloon catheter. The expanded balloon 9 compresses the stenosis 62 and thus increases the luminal diameter of the blood vessel 58.

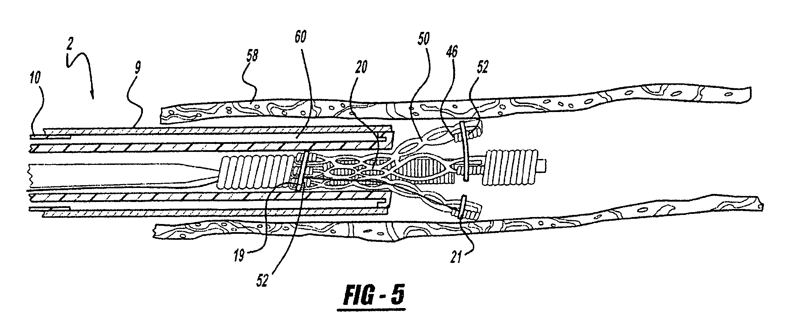

Figure 5 illustrates the balloon 9 in a deflated configuration and the balloon catheter 2 is moved proximally exposing the distal portion of the stent 20. Initially the distal portion of the stent is held in compression by the actuatable retaining ring 21. When an electrical current is applied to the resistive heating element 27 the distal actuatable retaining ring 21 begins to yield and then becomes severed as illustrated. At this point the distal portion of the stent 20 expands into contact with the vessel wall. After expansion of the distal portion of the stent 20, it is determined that the stent has been improperly positioned, the partially expanded stent may be simply withdrawn back into the balloon catheter 2. The catheter 2 may then be moved to a new position and the stent may again be moved distally thereby permitting the distal portion of the stent to expand. Since the actuatable retaining ring 21 was severed during the initial placement of the stent, the distal end of the stent will automatically expand into contact with the inside wall of the vessel.

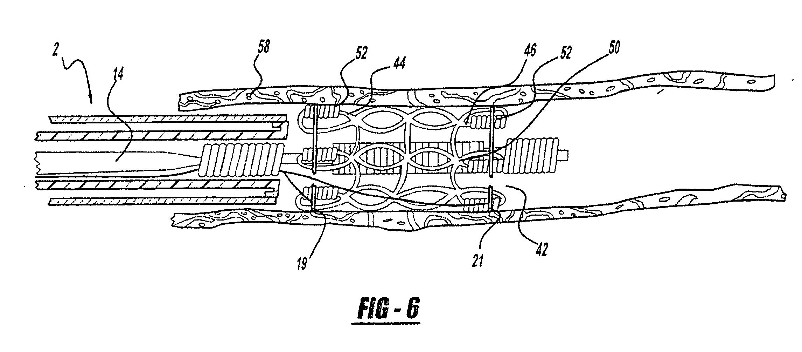

If on the other hand the distal portion of the stent is properly positioned, the balloon catheter 2 is again moved proximally, as illustrated in Figure 6 to also expose the proximal portion of the stent. An electrical current may then be applied to the proximal heating element 25 to thereby cause the heating element 25 to begin heating, which in turn, causes the proximal actuatable retaining ring 19 to yield and become severed. Once the retaining ring 19 severs, the proximal portion of the stent 20 expands into contact with the inside wall of the vessel.

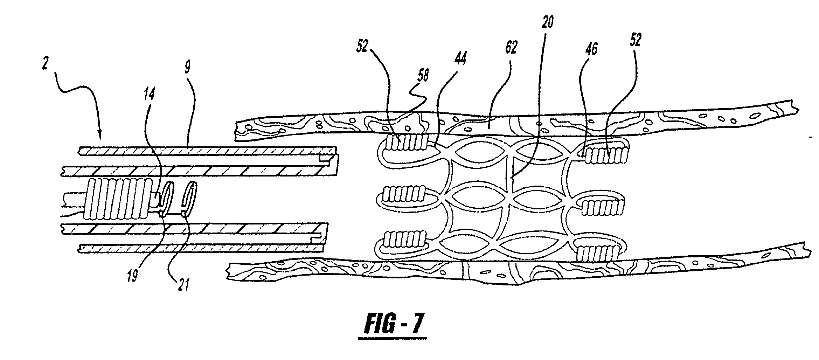

Figure 7 illustrates elongated core wire 14 entirely withdrawn from the stent 20 and into the lumen of the balloon catheter 2. The elongated core wire 14 carries with it into the lumen the severed actuatable retaining rings 19, 21.

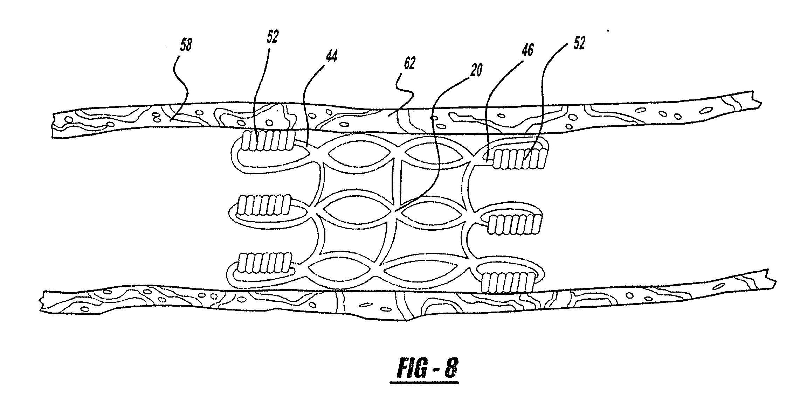

Figure 8 illustrates the self-expanding stent 20 expanded into the interior of the vessel so as to serve as a scaffold for maintaining patentcy of the vessel.

A novel system has been disclosed in which a self-expanding stent is mounted on an elongated core member and is slidably disposed within a balloon catheter. Although a preferred embodiment of the present invention has been described, it is to be understood that various modifications may be made by those skilled in the art without departing from the scope of the claims. For example, the actuatable retaining rings 19, 21 could be formed of a material which would be severed by the process of electrolysis, through a chemical reaction or through another form of electrical activation. Such alternative designs would not depart from the scope of the claims which follow.

## Claims

1. A self-expanding stent (20) and stent delivery system comprising:
an elongated core member (14) having proximal and distal portions including a proximal cylindrical member (16) disposed at the distal portion of said elongated core member (14), and a distal cylindrical member (18) disposed at the distal portion of said elongated core member (14) and positioned distally of said proximal cylindrical member (16) and being spaced apart from said proximal cylindrical member (16) to define a gap having a predetermined length;
a self-expanding stent (20) having a proximal end which is distal to said proximal cylindrical member (16), said self-expanding stent (20) being comprised of a small diameter skeletal tubular member having an outer cylindrical surface which defines a thin wall, said wall of said skeletal tubular member including a plurality of cells which are formed by a plurality of interconnected strut members (44, 46), and an anchor member (52) placed on one of said plurality of strut members (44, 46) at said proximal end (48) of said stent (20) and having a length less than the length of the gap between the proximal cylindrical member (16) and the distal cylindrical member (18), and said self-expanding stent (20) being mounted and compressed onto said elongated core member (14) such that said anchor member (52) at said proximal end (48) of said stent (20) is interlocked within said gap and between said proximal cylindrical member (16) and said distal cylindrical member (18) to thereby retain said stent (20) on said elongated core member (14); and,
a first actuatable retaining ring (19) is disposed around the outer cylindrical surface of said self-expanding stent (20) at said anchor member (52) for retaining said stent (20) onto said elongated core member (14) in a compressed state and said anchor member (52) in said gap, for upon actuation, releasing said self-expanding stent (20) to permit said anchor member (52) to move toward the vessel and the stent (20) to expand against the wall of the vessel and to permit the first actuated retaining ring (19) to be removed from the released stent (20).

2. A self-expanding stent and stent delivery system as defined in Claim 1, wherein said self-expanding stent (20) has proximal and distal sections (48, 50), and further comprising a second actuatable retaining ring (21) disposed around the distal portion of the stent (20).

3. A self-expanding stent (20) and stent delivery system as defined in Claim 1, wherein said first actuatable retaining ring (19) is formed of a material which when heated permits the compressed self-expanded stent (20) to expand into contact with the wall of a vessel.

4. A self-expanding stent (20) and stent delivery system as defined in Claim 3, including a heating element (25) positioned in proximity to said first actuatable retaining ring (19), and electrical conductors (28, 29) connected to said heating element (25) for, upon being energized, causing the temperature of said heating element (25) to increase thereby causing the temperature of the first actuatable retaining ring (19) to increase with the result that the first actuatable retaining ring (19) yields to permit the stent (20) to expand against the wall of a vessel.

5. A self-expanding stent (20) and stent delivery system as defined in Claim 3, in which the first actuatable retaining ring (19) is comprised of a polymeric material.

6. A self-expanding stent (20) and stent delivery system as defined in Claim 5, in which the first actuatable retaining ring (19) takes the form of a polymeric filament.

7. A self-expanding stent (20) and stent delivery system as defined in Claim 5, in which the first actuatable retaining ring (19) is comprised of a filament formed from a hot melt polymer.

8. A self-expanding stent (20) and stent delivery system as defined in Claim 4, in which the heating element (25) is an electrical resistive heating element.

## Patentansprüche

1. Selbstexpandierender Stent (20) und Stenteinbringungssystem, umfassend:
ein längliches Kemelement (14) mit proximalen und distalen Abschnitten, enthaltend ein proximales zylindrisches Element (16), das an dem distalen Abschnitt des länglichen Kernelements (14) angeordnet ist, und ein distales zylindrisches Element (18), das an dem distalen Abschnitt des länglichen Kernelements (14) angeordnet und distal von dem proximalen zylindrischen Element (16) positioniert und von dem proximalen zylindrischen Element (16) im Abstand angeordnet ist, um einen Spalt mit einer vorab festgelegten Länge zu definieren;
einen selbstexpandierenden Stent (20) mit einem proximalen Ende, das sich distal zum proximalen zylindrischen Element (16) befindet, wobei der selbstexpandierende Stent (20) aus einem Rohrskelettelement mit kleinem Durchmesser mit einer äußeren zylindrischen Fläche, die eine dünne Wand definiert, wobei die Wand des Rohrskelettelements eine Vielzahl von Zellen enthält, die aus einer Vielzahl von miteinander verbundenen Strebenelementen (44, 46) gebildet sind, und aus einem Ankerelement (52) besteht, das auf einem der Vielzahl von Strebenelementen (44, 46) an dem proximalen Ende (48) des Stents (20) plaziert ist und eine Länge aufweist, die geringer als die Länge des Spalts zwischen dem proximalen zylindrischen Element (16) und dem distalen zylindrischen Element (18) ist, wobei der selbstexpandierende Stent (20) auf dem länglichen Kemelement (14) so montiert und komprimiert ist, daß das Ankerelement (52) an dem proximalen Ende (48) des Stents (20) in dem Spalt und zwischen dem proximalen zylindrischen Element (16) und dem distalen zylindrischen Element (18) verriegelt ist, um dadurch den Stent (20) auf dem länglichen Kemelement (14) zu halten; und
einen ersten betätigbaren Haltering (19), der um die äußere zylindrische Fläche des selbstexpandierenden Stents (20) an dem Ankerelement (52) zum Halten des Stents (20) auf dem länglichen Kemelement (14) in einem komprimierten Zustand und des Ankerelements (52) in dem Spalt und bei Betätigung zum Freigeben des selbstexpandierenden Stents (20), so daß sich das Ankerelement (52) in Richtung auf das Gefäß bewegen kann und sich der Stent (20) an der Wand des Gefäßes expandieren kann und der erste betätigte Haltering (19) von dem freigegebenen Stent (20) entfernt werden kann, angeordnet ist.

2. Selbstexpandierender Stent und Stenteinbringungssystem nach Anspruch 1, **dadurch gekennzeichnet, daß** der selbstexpandierende Stent (20) proximale und distale Abschnitte (48, 50) aufweist und ferner einen zweiten betätigbaren Haltering (21) aufweist, der um den distalen Abschnitt des Stents (20) angeordnet ist.

3. Selbstexpandierender Stent (20) und Stenteinbringungssystem nach Anspruch 1, **dadurch gekennzeichnet, daß** der erste betätigbare Haltering (19) aus einem Material gebildet ist, das bei Erwärmung ermöglicht, daß der komprimierte selbstexpandierende Stent (20) in Kontakt mit der Wand eines Gefäßes expandiert.

4. Selbstexpandierender Stent (20) und Stenteinbringungssystem nach Anspruch 3, enthaltend ein Heizelement (25), das in der Nähe des ersten betätigbaren Halterings (19) positioniert ist, und elektrische Leiter (28, 29), die mit dem Heizelement (25) verbunden sind, um bei Speisung mit Strom zu bewirken, daß die Temperatur des Heizelements (25) ansteigt, und dadurch zu bewirken, daß die Temperatur des ersten betätigbaren Halterings (19) mit der Folge ansteigt, daß der erste betätigbare Haltering (19) dazu führt, daß der Stent (20) an der Wand eines Gefäßes expandieren kann.

5. Selbstexpandierender Stent (20) und Stenteinbringungssystem nach Anspruch 3, **dadurch gekennzeichnet, daß** der erste betätigbare Haltering (19) aus einem polymeren Material besteht.

6. Selbstexpandierender Stent (20) und Stenteinbringungssystem nach Anspruch 5, **dadurch gekennzeichnet, daß** der erste betätigbare Haltering (19) die Gestalt eines polymeren Filaments aufweist.

7. Selbstexpandierender Stent (20) und Stenteinbringungssystem nach Anspruch 5, **dadurch gekennzeichnet, daß** der erste betätigbare Haltering (19) aus einem Filament besteht, das aus einem Schmelzpolymer gebildet ist.

8. Selbstexpandierender Stent (20) und Stenteinbringungssystem nach Anspruch 4, **dadurch gekennzeichnet, daß** das Heizelement (25) ein Element für elektrisches Widerstandsheizen ist.

## Revendications

1. Endoprothèse auto-dilatable (20) et système de mise en place d'endoprothèse comprenant :
◆ un élément central allongé (14) ayant des parties proximale et distale comprenant un élément cylindrique proximal (16) disposé au niveau de la partie distale dudit élément central allongé (14), et un élément cylindrique distal (18) disposé au niveau de la partie distale dudit élément central allongé (14) et positionné de manière distale par rapport audit élément cylindrique proximal (16), et étant espacé dudit élément cylindrique proximal (16) pour définir un intervalle d'une longueur prédéterminée ;
◆ une endoprothèse auto-dilatable (20) ayant une extrémité proximale qui est distale par rapport audit élément cylindrique proximal (16), ladite endoprothèse auto-dilatable (20) comportant un élément de squelette tubulaire de petit diamètre ayant une surface cylindrique extérieure qui définit une mince paroi, ladite paroi dudit élément de squelette tubulaire comprenant une pluralité de cellules qui sont formées d'une pluralité d'éléments formant des branches entrelacées (44, 46) et un élément d'ancrage (52) placé sur une de ladite pluralité d'éléments formant des branches (44, 46) à ladite extrémité proximale (48) de ladite endoprothèse (20) et ayant une longueur inférieure à la longueur de l'intervalle entre l'élément cylindrique proximal (16) et l'élément cylindrique distal (18), et ladite endoprothèse auto-dilatable (20) étant montée sur, et comprimée contre, ledit élément central allongé (14) de sorte que ledit élément d'ancrage (52) à ladite extrémité proximale (48) de ladite endoprothèse (20) est bloqué dans ledit intervalle, et entre ledit élément cylindrique proximal (16) et ledit élément cylindrique distal (18), pour retenir ainsi ladite endoprothèse (20) sur ledit élément central allongé (14) ; et
◆ une première bague de retenue actionnable (19) est disposée autour de la surface cylindrique extérieure de ladite endoprothèse auto-dilatable (20) au niveau dudit élément d'ancrage (52) pour retenir ladite endoprothèse (20) sur ledit élément central allongé (14) dans un état comprimé et ledit élément d'ancrage (52) dans ledit intervalle, afin, lorsqu'elle est actionnée, de libérer ladite endoprothèse auto-dilatable (20), de permettre audit élément d'ancrage (52) de se déplacer vers le vaisseau, et à l'endoprothèse (20) de se dilater contre la paroi du vaisseau, et pour permettre à la première bague de retenue actionnée (19) d'être enlevée de l'endoprothèse ainsi libérée (20).

2. Endoprothèse auto-dilatable et système de mise en place d'endoprothèse selon la revendication 1, dans lequel ladite endoprothèse auto-dilatable (20) a des sections proximale et distale (48, 50), et comprenant en outre une seconde bague de retenue actionnable (21) disposée autour de la partie distale de l'endoprothèse (20).

3. Endoprothèse auto-dilatable (20) et système de mise en place d'endoprothèse selon la revendication 1, dans lequel ladite première bague de retenue actionnable (19) est formée d'un matériau qui, une fois chauffé, permet à l'endoprothèse auto-dilatée comprimée (20) de se dilater au contact de la paroi d'un vaisseau.

4. Endoprothèse auto-dilatable (20) et système de mise en place d'endoprothèse selon la revendication 3, comprenant un élément chauffant (25) positionné à proximité de ladite première bague de retenue actionnable (19), et des conducteurs électriques (28, 29) reliés audit élément chauffant (25) afin, une fois alimentés, d'amener la température dudit élément chauffant (25) à augmenter, en amenant ainsi la température de la première bague de retenue actionnable (19) à augmenter, ce qui fait que la première bague de retenue actionnable (19) cède pour permettre à l'endoprothèse (20) de se dilater contre la paroi d'un vaisseau.

5. Endoprothèse auto-dilatable (20) et système de mise en place d'endoprothèse selon la revendication 3, dans lesquels la première bague de retenue actionnable (19) se compose d'un matériau polymère.

6. Endoprothèse auto-dilatable (20) et système de mise en place d'endoprothèse selon la revendication 5, dans lesquels la première bague de retenue actionnable (19) prend la forme d'un filament polymère.

7. Endoprothèse auto-dilatable (20) et système de mise en place d'endoprothèse selon la revendication 5, dans lesquels la première bague de retenue actionnable (19) comprend un filament formé d'un polymère thermofusible.

8. Endoprothèse auto-dilatable (20) et système de mise en place d'endoprothèse selon la revendication 4, dans lesquels l'élément chauffant (25) est un élément chauffant à résistance électrique.
